# EUROPÄISCHE PATENTSCHRIFT

(11) **EP 0 667 793 B1**
(45) Veröffentlichungstag und Bekanntmachung des Hinweises auf die Patenterteilung: **08.01.1997**
(21) Anmeldenummer: 93924556.9
(22) Anmeldetag: 30.10.1993
(51) Int. Cl.: A61M 15/00

(54) **VERFAHREN UND VORRICHTUNG ZUM ERZEUGEN EINES AEROSOLS AUS EINER PULVERFÖRMIGEN SUBSTANZ**
METHOD AND DEVICE FOR PRODUCING AN AEROSOL FROM A SUBSTANCE IN POWDER FORM
PROCEDE ET DISPOSITIF PERMETTANT DE PRODUIRE UN AEROSOL A PARTIR D'UNE SUBSTANCE PULVERULENTE

(30) Priorität: 06.11.1992 DE 4237568
(43) Veröffentlichungstag der Anmeldung: 23.08.1995
(73) Patentinhaber: KÖHLER, Dieter, Prof. Dr. med., 57392 Schmallenberg-Winkhausen (DE)
(72) Erfinder: KÖHLER, Dieter, Prof. Dr. med., 57392 Schmallenberg-Winkhausen (DE)
(74) Vertreter: Lempert, Jost, Dipl.-Phys. Dr. rer.nat.
(86) Internationale Anmeldenummer: EP9303033
(87) Internationale Veröffentlichungsnummer: WO9411043

(56) Entgegenhaltungen:
- EP-A- 0 237 507
- WO-A-92/04066
- US-A- 4 534 343

## Beschreibung

Die Erfindung betrifft ein Verfahren nach dem Oberbegriff des Anspruchs 1 und eine Vorrichtung nach dem Oberbegriff des Anspruchs 11.

Zur Behandlung von Erkrankungen der Atemwege werden vorzugsweise Arzneistoffe mittels Inhalation appliziert, weil dadurch der Wirkstoff in relativ hoher Konzentration auf den Atemwegen deponiert wird, während Effekte in Folge der Verteilung des Arzneistoffs im ganzen Körper, sogenannte systemische Effekte, relativ gering bleiben. Ungeachtet dessen eignet sich die Inhalation eines Arzneistoffs auch zur systemischen Therapie.

Die Herstellung eines geeigneten Aerosols mit wirkungsvoll inhalierbaren, d.h. relativ kleinen Partikeln bereitet zum Teil erhebliche Schwierigkeiten, insbesondere wenn von einer pulverförmigen Substanz ausgegangen wird. Aus diesem Grund sind zahlreiche Verfahren und Vorrichtungen entwickelt worden, die auf unterschiedliche Art versuchen, ein solches gut inhalierbares Aerosol herzustellen.

Ungeachtet der verschiedenen Möglichkeiten sollte bei Inhalationsgeräten der Einsatz von Treibgasen, insbesondere solcher, deren Umweltschädlichkeit erkannt wurde, vermieden werden.

Aus der DE-A1-40 27 390 ist ein treibgasfreies Inhalationsgerät bekannt, bei dem die medizinische Substanz in Pulverform in einem Vorratsbehälter aufgenommen ist. Mit einer Dosiereinrichtung wird eine Dosis der Substanz entnommen, vor ein Mundstück gebracht und von dort vom Patienten mit Hilfe aktiver Einatmung inhaliert, wobei die Substanz von dem sich durch das Einatmen ausbildenden Luftstrom mitgerissen wird. Um inhalierbare Partikel der pulverförmigen Substanz zu erzielen, ist ein Luftraum zur Verteilung der Substanz in dem sich ausbildenden Luftstrom vorgesehen. Die Partikelgröße hängt bei diesem Inhalationsgerät in starkem Maße von der Stärke und der Art des Einatmens ab, so daß hinreichend kleine Partikel der dosierten Substanz nicht in jedem Fall gewährleistet sind. Für den Einsatz in dem bekannten Inhalationsgerät wird der Arzneistoff - eventuell unter Zumischung von Hilfsstoffen - zu einem Preßling verdichtet. Von dem Preßling wird mit Hilfe einer bewegten Kante oder Bürste Pulver abgetragen, das der Atemluft des Patienten beigemischt wird. Das Verfahren ist für die Massenproduktion nicht geeignet, weil keine Möglichkeit besteht, die Preßlinge aus Arznei- und Hilfsstoff mit homogener Härte herzustellen.

Aus der DE-A-40 27 391 ist ein weiteres treibgasfreies Inhalationsgerät mit ähnlichem Aufbau bekannt. Um die eingeatmete Substanzmenge reproduzierbar zu machen und gleichzeitig inhalierbare Partikel zu erreichen, wird aber ein in einem Zylinder gespeichertes Luftvolumen in Reaktion auf das Einatmen selbsttätig freigegeben und zu einer Düse geführt, wodurch die dosierte Substanz durch den sich ausbildenden aktiven Luftstrom in den Rachen des Patienten geblasen wird. Problematisch ist bei der Atemzugtriggerung aber der erhebliche Aufwand, der für eine exakte, durch den Atemzug ausgelöste Freigabe des Luftvolumens erforderlich ist.

Bekannte Verfahren und Vorrichtungen haben jedoch in der Regel den Nachteil, daß ein bezüglich Partikelspektrum sowie Nebeldichte ungenaues Aerosol entsteht. Grundsätzlich kommt es beim Anblasen zu einer Verwirbelung von einer erheblichen Luftmenge, die wesentlich größer ist als das Anblasvolumen. Diese Luftmenge muß normalerweise in einem entsprechenden Behälter abgefangen werden, wenn sie der Patient nicht inhaliert. Inhaliert sie der Patient direkt, so ist die intrabronchiale Deposition außerordentlich unterschiedlich, da abhängig vom Inhalationsfluß das Partikelspektrum vorwiegend durch Impaktion stark modifiziert wird.

Beim Anblasen wird die beschleunigte Luft durch die vorhandene abgebremst. Die kinetische Energie, die auf das Aerosol prallt, ist dadurch nicht hoch.

Wenn man das angeblasene Aerosolvolumen in einen Behälter zwischenspeichert, damit der Patient immer ein gleiches Partikelspektrum inhaliert, sind große Volumina erforderlich. Bei Dosier-Aerosolen werden oft Spacer mit Volumen von 300-800 ml als Vorsatzgeräte angewendet.

Ein weiteres Inhalationsgerät mit aktivem Luftstrom ist aus der WO-A-90/07351 bekannt. Der Luftstrom wird von einer Druckgasquelle bereitgestellt oder mit Hilfe einer Kolben/Zylinder-Anordnung erzeugt und in eine Düsenanordnung mit sich änderndem Querschnitt geführt, um die Strömungsgeschwindigkeit des Luftstroms gezielt zu erhöhen. Im Bereich der höchsten Strömungsgeschwindigkeit mündet in der Düsenanordnung ein Kanal in den Strömungsweg und stellt eine Verbindung zu einem Vorratsbehälter für die pulverförmige Substanz her. Die Substanz wird von dem aktiven Luftstrom angesaugt und in eine Mischkammer befördert, wo eine möglichst gute Verteilung der Partikel stattfinden soll. Bei diesem Inhalationsgerät ist zu beachten, daß die Bereitstellung des Aerosols völlig unabhängig vom Einatmen des Patienten erfolgt und ebenfalls ein aktiver Luftstrom in die Atemwege des Patienten gerichtet ist, so daß eine gute Verteilung vor und während des Einatmens nicht erwartet werden kann.

Aus der WO 92/04066 ist eine Inhalationsvorrichtung bekannt, die zum Inhalieren einer aktiven pulverförmigen oder flüssigen Substanz vorgesehen ist. Bei dem bekannten Gerät befindet sich die Gesamtmenge der Substanz in einem auf einem Mundstück aufsitzenden herkömmlichen Inhalator, der also einen Vorratsraum bzw. ein Vorratsgefäß bildet. Hiervon zu unterscheiden ist ein Verwirbelungsraum in einem anderen Teil des Gerätes, in den eine zu inhalierende Dosis an wirksamer Substanz aus dem Inhalator durch Herstellung des Unterdrucks im Verwirbelungsraum eingesaugt wird. Das bekannte Gerät ist insofern aufwendig und kompliziert, da es einerseits Raum für den Vorratsbehälter und andererseits für den Verwirbelungsraum benötigt.

Bei dem bekannten Gerät weist der Verwirbelungsraum einen Kolben auf, der zwischen einer unteren und einer oberen Position, in der er durch ein lösbares Blockierglied gehalten werden kann, bewegbar ist. Wenn das Blockierglied gelöst wird, wird der Kolben unter Wirkung einer Feder in seine untere Position bewegt, wodurch das wirksame Pulver oder die flüssige Substanz in dispergierter Form aus dem konventionellen Inhalator, der mit dem Verwirbelungsraum in Verbindung steht, eingesaugt wird. Ein Patient kann Luft und in dieser dispergierte aktive Substanz durch ein Mundstück über Einwegventile ansaugen.

Der Ablauf ist also hier derart, daß gleichzeitig mit der Erzeugung von Unterdruck schon dispergierte aktive Substanz, also aerosolförmige Substanz, in den Verwirbelungsraum eingesaugt wird. Es wird also gleichzeitig Unterdruck erzeugt und Gas eingesaugt. Demgemäß wird die wirksame Substanz zusammen mit dem Gas in den Verwirbelungsraum eingesaugt. Das Aerosol muß von einem Patienten aktiv inhaliert werden, d.h. es muß aus dem Raum herausgesaugt werden.

Der Erfindung liegt die Aufgabe zugrunde, ein Verfahren und eine Vorrichtung zur Erzeugung eines Aerosols aus einer pulverförmigen Substanz anzugeben, mit dem bzw. mit der ein Aerosol hoher Dichte mit inhalierbaren, lungengängigen, insbesondere relativ kleinen Partikeln der Substanz zur Inhalation bereitgestellt wird, ohne daß das Einatmen des Aerosols störend beeinflußt wird.

Gelöst wird diese Aufgabe durch ein Verfahren gemäß Patentanspruch 1 und durch eine Vorrichtung gemäß Patentanspruch 11.

Bei der Erfindung erfolgt die Verwirbelung der pulverförmigen Substanz in einem definierten, abgeschlossenen Verwirbelungsraum durch Einströmen eines Gases, insbesondere Luft in diesen. Durch die mechanische Erzeugung des Unterdrucks mittels eines Kolbens oder einer Pumpe kann ein viel geringerer Druck erzeugt werden, als dies durch Saugen eines Patienten möglich wäre. Hierdurch wird dann eine wesentlich größere Einströmungsgeschwindigkeit möglich, die erst zu der gewünschten guten Zerstäubung der Substanzpartikel führt. Anschließend wird das hierdurch gebildete Aerosol, vorzugsweise ebenfalls eine definierte Menge bzw. ein definiertes Volumen bei Normaldruck, in den Innenraum eines Mundstücks in vorbestimmter Menge eingebracht, vorzugsweise indem im Verwirbelungsraum zunächst eine Druckerhöhung erfolgt und sodann durch Öffnen einer Austrittsöffnung die vorbestimmte Menge des Aerosols bis zum Druckausgleich ausströmt; anschließend, nach Schließen der Austrittsöffnung des Verwirbelungsraumes, kann der Patient oder Benutzer das Aerosol aus dem Mundstück einatmen.

Es wird also in dem Verwirbelungsraum zunächst ein Unterdruck erzeugt und erst anschließend Gas in den Verwirbelungsraum eingesaugt. Dabei wird die im Verwirbelungsraum enthaltene pulverförmige Substanz in diesem verwirbelt.

Durch die Erfindung wird die Freisetzung und Agglomeration eines mikronisierten Pulvers in einem kleinen Volumen möglich. Das beim Anblasen von Pulver normalerweise entstehende erhebliche Strömungsvolumen und der damit verbundene Überschuß an Luft, der oft ein Vielfaches der benötigten Anblasluft beträgt, werden durch die Erfindung vermeidbar. Es sind daher keine großvolumigen Auffangbehälter mehr zwingend erforderlich. Das kleine Volumen ist sehr genau definierbar und führt zu einer Vereinfachung der Dosierung. Es bildet sich ein stabiles Aerosol, da größere Partikel sofort durch Sedimentation abgeschieden werden. Ungewöhnlich hohe Pulverdichten bis zu 10 mg/l sind mit Hilfe der Erfindung erreichbar und damit sind entsprechend kleine Dosieraerosole möglich.

Beim erfindungsgemäßen Verfahren strömt Luft in ein definiertes Volumen. Die hineinströmende Luft füllt also immer das gleiche Volumen. Sie führt zur Desagglomeration und Zerstäubung des Pulvers in einer außerordentlich hohen Konzentration. Dagegen wird - wie oben schon erwähnt - beim Anblasen eines Pulvers, um es in eine Aerosolform überzuführen, durch das Verwirbelungsvolumen das Aerosol verdünnt. Bei dem erfindungsgemäßen Verfahren entsteht eine so hohe Aerosoldichte, daß damit die Grenzkonzentration erreicht wird, die die Luft noch tragen kann. Infolgedessen fallen insbesondere die gröberen Partikel rasch (innerhalb von Sekunden) aus und es resultiert eine stabile Nebeldichte (Aerosoldichte) mit etwa gleichem Partikelspektrum. Diese hohe Nebeldichte kann durch Anblasen nie erreicht werden. Die Nebeldichte ist infolge der "Übersättigung" der Luft durch das Aerosol unabhängig von der Füllmenge. Dies ist bei den bekannten Verfahren nicht der Fall. Gerade das hebt das erfindungsgemäße Verfahren und die erfindungsgemäße Vorrichtung von dem Stand der Technik ab. Das kleine Volumen hat auch noch den großen Vorteil der Handlichkeit.

Gemäß bevorzugter Ausgestaltungen ist vorgesehen, daß vor dem Einströmen eines Gases in den Verwirbelungsraum in diesem ein Druck von weniger als 100 Pa, wie etwa 70 Pa, vorzugsweise sogar weniger als 50 Pa, wie beispielsweise 30 Pa, erzeugt wird, wobei dies in vorteilhafter Weise dadurch erreicht wird, daß der Verwirbelungsraum in abgeschlossenem Zustand zur Herstellung eines Unterdrucks in ihm vergrößerbar ist und daß nach Öffnen der Öffnung das Gas in den Verwirbelungsraum aufgrund des in diesem erzeugten Unterdrucks einströmt. Hierdurch werden Strömungsgeschwindigkeiten im Verwirbelungsraum von mehr als 200 m/sec möglich, die erst zu der gewünschten Desagglomeration durch Überwindung der Adhäsion der Teilchen führen. Hierdurch wird ein lungengängiger Partikelanteil von 90 % und mehr erzeugt.

Bei einem Unterdruck strömt die Luft ungehindert auf die pulverförmige Substanz. Die Geschwindigkeit wird nur durch die Massenträgheit der Luft und die Höhe des Vakuums begrenzt. Die Erzeugung der Nebeldichte ist sehr hoch.

Weitere bevorzugte Ausgestaltungen sehen vor, daß der Unterdruck in dem die Substanz enthaltenden Verwirbelungsraum durch Vergrößern des Verwirbelungsraumes erzeugt wird und daß das Aerosol aus dem das Aerosol enthaltenden Verwirbelungsraum durch Herausdrücken ausgebracht wird sowie insbesondere daß das in den Verwirbelungsraum einströmende Gas Umgebungsluft ist.

Während grundsätzlich vorgesehen sein kann, daß die Verwirbelung in einem Vorratsraum für die Substanz erfolgt, sieht eine äußerst bevorzugte Ausgestaltung vor, daß eine definierte Menge der Substanz in den Verwirbelungsraum zur Verwirbelung derselben eingebracht wird.

Weitere bevorzugte Ausgestaltungen sehen vor, daß ein mit dem Verwirbelungsraum verbindbares Mundstück während des Eindrückens des Aerosols in das Mundstück bis gegebenenfalls auf einen Auslaß des Mundstücks für das spätere Einatmen durch den Patienten gegenüber der Umgebung absperrbar ist und/oder daß ein mit dem Verwirbelungsraum verbindbares Mundstück nach Eindrücken des Aerosols in das Mundstück auch bei an diesem angesetzten Mund des Benutzers zur Umgebung geöffnet ist.

Während zum Verwirbeln eine turbulente Luftströmung erzeugt wird und hierzu auch gewünscht ist, soll die Luft in und durch den Mund des Patienten sowie seine Luftröhre bis in die Lunge möglichst laminar strömen. Eine bevorzugte Weiterbildung sieht daher vor, daß das Aerosol weitgehend laminar in das Mundstück strömt, wobei dies vorrichtungsmäßig dadurch erreicht wird, daß zwischen der Austrittsöffnung des Verwirbelungsraums und dem Inneren eines Mundstücks ein Strömungsgleichrichter angeordnet ist. Der Gleichrichter ist insbesondere als Siebplatte ausgebildet.

Weitere äußerst bevorzugte Ausgestaltungen der Erfindung zeichnen sich dadurch aus, daß der Verwirbelungsraum im Vorratsraum für die Substanz ausgebildet ist. Weiterhin kann vorgesehen sein, daß zwischen dem Innenraum eines Mundstücks und der Umgebung eine verschließbare Öffnung vorgesehen ist, die beim Öffnen der Austrittsöffnung des Verwirbelungsraums zum Ausströmen des Aerosols aus dem Verwirbelungsraum geschlossen ist und/oder daß zwischen dem Inneren eines Mundstücks und der Umgebung eine verschließbare Öffnung vorgesehen ist, die nach Schließen der Austrittsöffnung des Verwirbelungsraumes geöffnet ist. Während die Öffnung zum Inneren des Mundstücks einerseits und die Auslaßöffnung des Verwirbelungsraumes unabhängig in geeigneter Weise gesteuert werden könnten, sieht eine bevorzugte Ausgestaltung vor, daß ein Verschlußorgan für die Austrittsöffnung des Verwirbelungsraums und ein Verschlußteil für die Öffnung zwischen dem Inneren des Mundstücks und der Umgebung miteinander verbunden sind, wobei insbesondere Verschlußorgan und Verschlußteil starr miteinander verbunden sind.

Um einerseits ein Öffnen der Ein- und Auslaßöffnung zum Verwirbelungsraum erst nach Herstellen eines Unterdrucks ohne zusätzliche Betätigungsvorgänge zu erreichen, andererseits das Ausströmen des Gases nach Öffnen der Austrittsöffnung mit einem vorbestimmten Volumen durch vorherigen Aufbau eines Überdrucks zu erzielen, sehen weitere bevorzugte Ausgestaltungen vor, daß das Verschlußteil für die Austrittsöffnung des Verwirbelungsraumes mit einem in diesem bewegbaren Kolben durch einen Faden verbunden ist und daß ein das Volumen des Verwirbelungsraumes verändernder Kolben mit einem mit einem Verschlußorgan einer Austrittsöffnung des Verwirbelungsraums zusammenwirkenden Stößel zum Verschließen der Austrittsöffnung verbunden ist.

Damit die Bewegungen des Kolbens im Zylinder nicht durch an der Innenseite der Zylinderwandung haftendes Pulvermaterial behindert wird, ist in bevorzugter Ausgestaltung vorgesehen, daß die pulverförmige Substanz und der Verwirbelungsraum durch einen im Inneren des Zylinders angeordneten, flexiblen Mantel umgeben sind, der einerseits am Kolben, andererseits am Zylinder, nahe dessen dem Kolben abgewandten Ende, also nahe der Ein- und Auslaßöffnung festgelegt ist.

In weiterer Ausbildung kann vorgesehen sein, daß dem Verschlußorgan ein am Zylinder festgelegter Abstreifring zugeordnet ist.

Da bei einer erfindungsgemäßen Vorrichtung eine vorgegebene Substanz vorhanden ist, kann nur eine bestimmte Anzahl von Aerosolen erzeugt werden. Damit das Leeren des Zylinders festgestellt werden kann, ist ein Zählwerk vorgesehen. Dieses ist in bevorzugter Ausgestaltung derart gebildet, daß an einer Betätigungsstange des Kolbens im Zylinder eine flexible Nase angeordnet ist, die auf ein kleines Zahnrad oder ein Ritzel wirkt, welches durch eine an einer Stirnseite ausgebildete Schnecke ein größeres Zahnrad antreibt, welches mit farblichen Markierungen versehen ist, die durch ein Fenster beobachtet werden können. Statt des Zahnrads kann grundsätzlich auch ein entsprechend mit Markierungen versehener Zahnriemen vorgesehen sein.

Gemäß einer anderen bevorzugten Ausgestaltung ist vorgesehen, daß eine in den Verwirbelungsraum führende Öffnung in dem Kolben ausgebildet ist, wobei insbesondere der Kolben mit einem äußeren zylindrischen Gehäuse über eine Kolbenstange und einen quer in dem Gehäuse angeordneten Steg starr verbunden ist und der Zylinder durch einen zylindrischen Zylinderkörper mit einem an den Kolben angepaßten Innendurchmesser gebildet ist und der Zylinder in dem Gehäuse derart verschiebbar ist, daß der Verwirbelungsraum vergrößerbar ist. In Weiterbildung kann dann ein Verschlußteil vorgesehen sein, das an einem Arm eines zweiarmigen, an der Kolbenstange gelagerten Hebels angebracht ist, wobei der Zylinderkörper eine Nase aufweist, die mit einem anderen Arm des Hebels derart zusammenwirkt, daß bei Erreichen der Stellung des Zylinderkörpers, in der der Verwirbelungsraum am größten ist, die Öffnung im Kolben freigegeben wird.

Eine weitere bevorzugte Ausgestaltung sieht vor, daß der Verwirbelungsraum durch einen Faltenbalg gebildet ist, der an seinen Stirnseiten mit Deckplatten abgeschlossen ist, und daß in der einen Deckplatte eine erste Ventileinrichtung für das Einströmen des Gases in den evakuierten Verwirbelungsraum vorgesehen ist, die durch eine Auslöseeinrichtung ausgelöst wird, wenn durch Ausdehnen des Faltenbalgs der Verwirbelungsraum am größten ist, wobei insbesondere eine zweite Ventileinrichtung vorgesehen ist, durch die beim Zusammendrücken des Faltenbalgs das im Verwirbelungsraum vorhandene Gas daraus entweichen kann, und gegebenenfalls eine dritte Ventileinrichtung vorgesehen ist, die ebenfalls von der Auslöseeinrichtung ausgelöst wird, wenn durch Ausdehnen des Faltenbalgs der Verwirbelungsraum am größten ist, und durch die das im Verwirbelungsraum erzeugte Aerosol ausbringbar ist. Darüber hinaus kann ein Atemrohr vorgesehen sein, an dem die erste und dritte Ventileinrichtung angeschlossen sind und das im Bereich zwischen den beiden Anschlußpunkten der Ventileinrichtungen eine Verengung aufweist, durch die die Strömungsgeschwindigkeit des Luftstroms beim Atmen durch das Atemrohr erhöht wird.

Durch die Erfindung wird ein Verfahren und eine Vorrichtung angegeben, bei der das Aerosol einen hohen inhalierbaren Wirkstoffanteil bei einer guten Dosierkonstanz aufweist. Es hat sich herausgestellt, daß der inhalierbare Wirkstoffanteil bei 90 % liegt, während er beim Stand der Technik nur bei 20 % liegt. Der Wirkungsgrad wird durch die Erfindung daher wesentlich erhöht; es kann wesentlich weniger Wirkstoff verwendet werden, da dieser besser ausgenutzt wird. Die Mengenvariation war wesentlich geringer als bei bekannten Verfahren und Vorrichtungen, nämlich lediglich bei 11 %, während der Variationskoeffizient bei bekannten Verfahren und Vorrichtungen ein Vielfaches dieses Wertes beträgt. Darüber hinaus wird, wie schon angedeutet, gegenüber dem Stand der Technik eine relativ enge Partikelgrößenverteilung von etwa 0,6 bis knapp unter 6 µm erreicht, während größere Partikel praktisch überhaupt nicht im Aerosol auftreten.

Im folgenden werden das erfindungsgemäße Verfahren und Ausführungsbeispiele der erfindungsgemäßen Vorrichtung anhand der beiliegenden Zeichnungen näher erläutert. Dabei zeigt:
- Fig. 1A bis 1D: ein erstes Ausführungsbeispiel der erfindungsgemäßen Vorrichtung und die einzelnen Schritte des erfindungsgemäßen Verfahrens;
- Figur 2: ein zweites Ausführungsbeispiel der erfindungsgemäßen Vorrichtung;
- Fig. 3A und 3B: ein drittes Ausführungsbeispiel der erfindungsgemäßen Vorrichtung;
- Fig. 4A: einen Längsschnitt durch eine weitere bevorzugte Ausführungsform der erfindungsgemäßen Vorrichtung bei geschlossenem Ventil;
- Fig. 4B: den Längsschnitt der Fig. 4A bei geöffnetem Ventil; und
- Fig. 4C: einen Schmitt durch das Zählwerk entlang A-B der Fig. 4A.

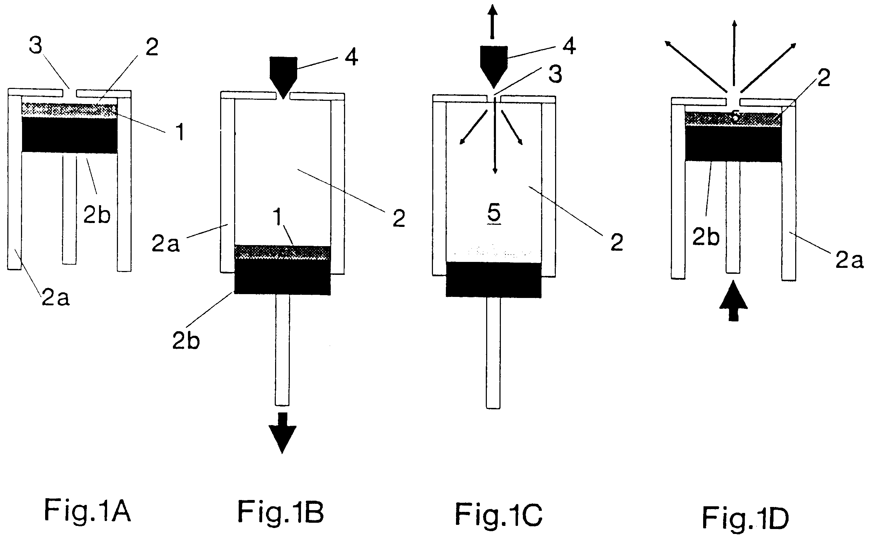

Anhand von Fig. 1 wird im folgenden zunächst das erfindungsgemäße Verfahren anhand eines ersten Ausführungsbeispiels der erfindungsgemäßen Vorrichtung erläutert. Dieses und weitere Ausführungsbeispiele der erfindungsgemäßen Vorrichtung werden im Anschluß daran näher beschrieben.

In Fig. 1A ist der erste Schritt des Verfahrens dargestellt, wonach eine definierte Menge der pulverförmigen Substanz 1 in einen abgeschlossenen Verwirbelungsraum 2 gebracht wird. Beim ersten Ausführungsbeispiel handelt es sich bei dem abgeschlossenen Verwirbelungsraum 2 um das Innere eines Zylinders 2a einer Anordnung aus Kolben und Zylinder. Das Einbringen der Substanz kann beispielsweie durch eine in dem Zylinder vorgesehene Austrittsöffnung 3, aber auch durch eine eigens dafür vorgesehene Öffnung oder auf andere Art erfolgen, etwa indem der Kolben 2b aus dem Zylinder 2a herausgezogen, die Substanz eingebracht und der Kolben wieder in den Zylinder eingesetzt wird.

In Fig. 1B ist der zweite Schritt des erfindungsgemäßen Verfahrens dargestellt, wonach in dem die Substanz enthaltenden Verwirbelungsraum 2 ein definiertes Vakuum erzeugt wird. Im Fall der Anordnung aus Kolben und Zylinder wird dieses Vakuum auf einfache Weise dadurch erreicht, daß der Kolben 2b aus der in Fig. 1A gezeigten, vorgeschobenen Stellung in die in Fig. 1B gezeigte, zurückgezogene Stellung gebracht wird. Die Austrittsöffnung 3 des Zylinders 2a wird dabei mit einem geeigneten Verschlußorgan 4 verschlossen. Bei der Erzeugung des Vakuums im Inneren des die Substanz enthaltenden Verwirbelungsraums 2 bleibt vorzugsweise die pulverförmige Substanz 1 im wesentlichen unbeeinflußt, wie in den Fig. 1A und 1B durch die Darstellung der Substanz 1 in der Nähe des Kolbens 2b angedeutet ist.

Der dritte Schritt des erfindungsgemäßen Verfahrens ist in Fig. 1C dargestellt. Danach wird es einem Gas, vorzugsweise der Umgebungsluft, gestattet, rasch, aber kontrolliert in den die Substanz enthaltenden Verwirbelungsraum 2 einzuströmen. Das heißt, es wird ein Luftstrom erzeugt, der in den Verwirbelungsraum rasch, aber kontrolliert einströmt. Bei dem gezeigten Ausführungsbeispiel geschieht dies durch Öffnen des Verschlußorgans 4 der Austrittsöffnung 3. Der sich einstellende Luftstrom ist in Fig. 1C durch Pfeile angedeutet; er verwirbelt die pulverförmige Substanz 1 und sorgt für eine gute Verteilung der Partikel in dem zur Verfügung stehenden Verwirbelungsraum 2, so daß das gewünschte Aerosol 5 erzeugt wird, das den Verwirbelungsraum nahezu vollständig ausfüllt. Die Verteilung der Partikel stellt sich unter sehr genau definierbaren Bedingungen ein, da durch die Größe und Gestalt des zur Verfügung stehenden Verwirbelungsraumes 2 und der Öffnung 3, durch die der Luftstrom in den Verwirbelungsraum gelangt, sowie durch deren Lage das Strömungsverhalten und damit die Verwirbelung der pulverförmigen Substanz in starkem Maße bestimmt werden kann.

So ist es beispielsweise möglich, die Agglomeration und Sedimentation größerer Partikel, die für die Inhalation weniger geeignet oder sogar ungeeignet sind, gezielt zu steuern. Dadurch wird die Ausbildung eines homogenen Aerosols mit konstanter Aerosoldichte weiter unterstützt.

Im vierten Schritt des erfindungsgemäßen Verfahrens wird, wie in Fig. 1D gezeigt, das erzeugte Aerosol 5 aus dem Verwirbelungsraum 2 ausgebracht und dadurch dem Patienten zur Inhalation bereitgestellt. Beim ersten Ausführungsbeispiel geschieht das Ausbringen des Aerosols durch einfaches Herausdrücken, indem der Kolben 2b wieder in seine vorgeschobene Stellung gebracht wird. Das Ausbringen kann aber auch dadurch geschehen, daß der Patient beim Einatmen das erzeugte Aerosol aus dem Verwirbelungsraum ansaugt. In jedem Fall kann das Ausbringen des Aerosols aber so geschehen, daß eine störende Beeinflussung des Inhalationsvorgangs beim Einatmen vermieden wird, da bei diesem Schritt keine Verwirbelung mehr erzielt werden muß. Das Aerosol 5 mit einer guten Verteilung der Partikel steht bereits vor dem Ausbringen im Verwirbelungsraum 2 zur Verfügung und füllt diesen gleichmäßig aus. Das Ausbringen kann daher vollständig an den Bedingungen ausgerichtet werden, die hinsichtlich der Inhalation zu berücksichtigen sind. Insbesondere kann eine störende Beeinflussung des Einatmens durch eine zu starke oder Zwangsluftströmung vermieden werden.

Um eine möglichst gute Anpassung der Öffnungen an ihre jeweilige Aufgabe durchführen zu können, können für das Einströmen der Luft in den evakuierten Verwirbelungsraum und für das Herausdrücken des Aerosols, ebenso wie für das Einbringen der Substanz in den Verwirbelungsraum auch getrennte Öffnungen vorgesehen werden.

Der Aufbau des ersten Ausführungsbeispiels der erfindungsgemäßen Vorrichtung ergibt sich bereits aus der obigen Schilderung des erfindungsgemäßen Verfahrens anhand dieses Ausführungsbeispiels.

Demnach umfaßt das erste Ausführungsbeispiel der Vorrichtung eine Anordnung aus einem Zylinder 2a und einem Kolben 2b, die gemeinsam einen Verwirbelungsraum 2 festlegen. Der Kolben 2b ist in dem Zylinder 2a beweglich angeordnet und kann zwischen einer vorgeschobenen und einer zurückgezogenen Stellung hin und her bewegt werden. Die Größe des Verwirbelungsraumes ist somit über die Position des Kolbens bestimmbar. Der Verwirbelungsraum 2 besitzt eine Öffnung 3, durch die die dosierte Substanz 1 in den Verwirbelungsraum 2 eingebracht wird, durch die ein die Substanz verwirbelndes Gas, vorzugsweise Umgebungsluft, einströmt und durch die das erzeugte Aerosol 5 herausgedrückt wird. Damit der Verwirbelungsraum 2 bei der Herstellung des definierten Vakuums abgeschlossen ist, ist ein Verschlußorgan 4 vorgesehen.

Um eine Beeinträchtigung der Bewegbarkeit des Kolbens 2b im Zylinder 2a auszuschließen, kann im Inneren des Zylinders ein zusätzlicher flexibler Behälter, etwa ein Faltenbalg oder ein dünnwandiger Kunststoffschlauch, zur Aufnahme der Substanz vorgesehen werden. Beim Erweitern des Verwirbelungsraumes, d.h. beim Verschieben des Kolbens 2b in die zurückgezogene Stellung, erweitert sich auch der flexible Behälter aufgrund des außen anliegenden Vakuums. Vorzugsweise kann der flexible Behälter auch an dem Kolben befestigt sein, so daß er mit dem Kolben mitbewegt wird.

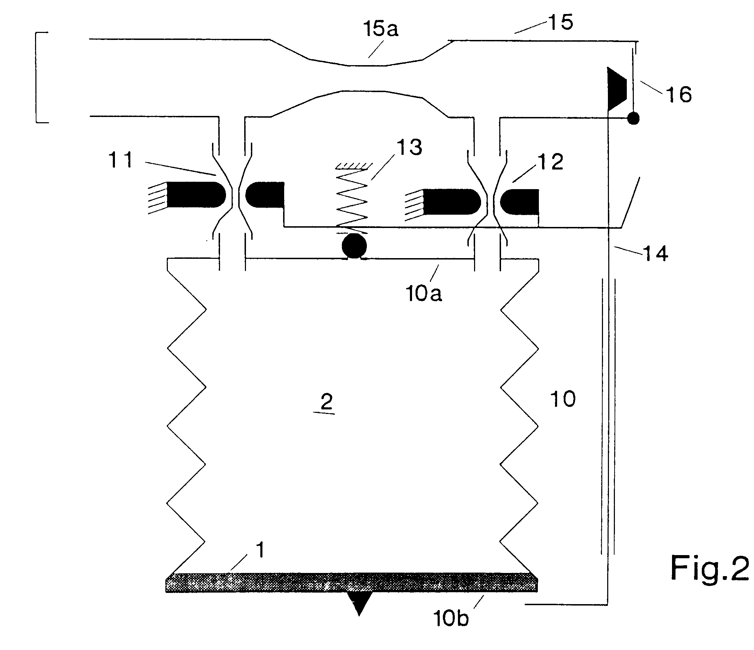

In Fig. 2 ist ein zweites Ausführungsbeispiel der erfindungsgemäßen Vorrichtung gezeigt, bei dem ein Faltenbalg 10 als der den Verwirbelungsraum festlegende Bestandteil verwendet wird. Der Faltenbalg 10 ist an seinen Stirnseiten mittels Deckplatten 10a und 10b abgeschlossen. In der Deckplatte 10a sind drei Ventile 11, 12 und 13 vorhanden, von denen die baugleichen Ventile 11 und 12 für das Einströmen der Luft in den evakuierten Verwirbelungsraum 2 und für das Ausbringen des Aerosols aus dem Verwirbelungsraum 2 vorgesehen sind und von denen das Ventil 13 ein Entweichen von Luft beim Zusammendrücken des Faltenbalgs 10 gestattet. Nachdem der Faltenbalg 10 wieder erweitert wurde, um ein Vakuum in dem die Substanz enthaltenden Verwirbelungsraum 2 zu erzeugen, wird als Auslöseeinrichtung für die Ventile 11 und 12 ein Schieber 16 betätigt, mit dem die beiden Ventile 11 und 12, die in Fig. 2 als Schlauchventile dargestellt sind, geöffnet werden.

Über die beiden Ventile 11 und 12 ist der Faltenbalg 10 und damit der Verwirbelungsraum 2 an ein Atemrohr 15 im Nebenschluß angeschlossen, durch das der Patient einatmet und das dafür an einem Ende mit einem Mundstück für den Patienten ausgestattet ist. An diesem Ende des Atemrohres 15 ist auch ein weiteres Ventil 16 vorgesehen, das ein Hineinblasen in das Atemrohr verhindert. Das Atemrohr 15 besitzt eine Verengung 15a, damit zufolge des Druckabfalls ein ausreichender Luftstrom durch den Verwirbelungsraum 2 strömt. Nicht dargestellt sind Vorrichtungen, die verhindern, daß die Substanz 1, insbesondere eine pulverförmige Substanz, in die Ventile gelangt und dadurch die Funktionsfähigkeit der Ventile beeinträchtigt bzw. die Substanz 1 über das Atemrohr 15 oral aufgenommen wird.

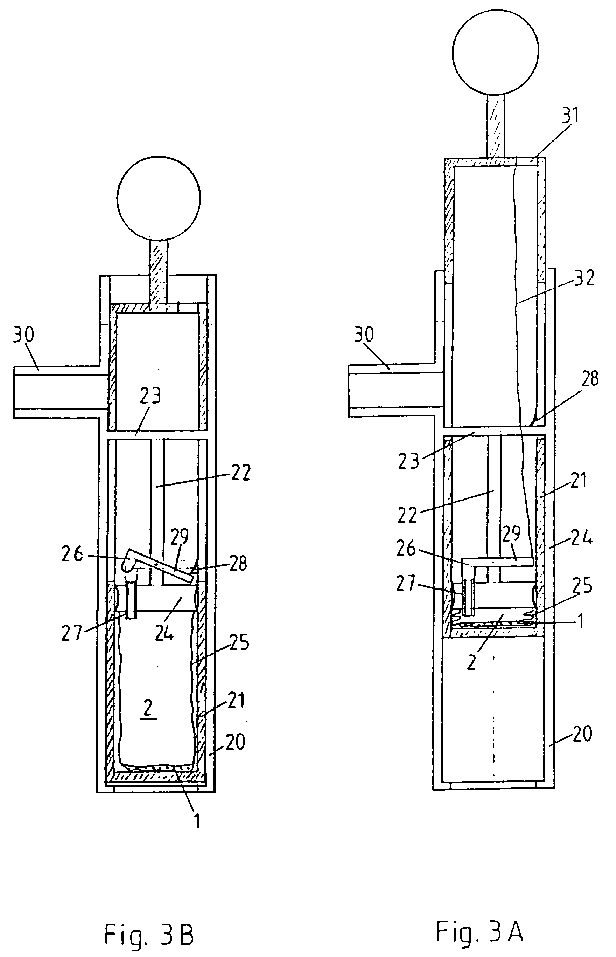

Ein drittes Ausführungsbeispiel der erfindungsgemäßen Vorrichtung ist in den Fig. 3A und 3B dargestellt. Die Vorrichtung besteht aus einem äußeren zylindrischen Gehäuse 20 und einem inneren zylindrischen Zylinderkörper 21, der in dem Gehäuse zwischen den zwei Stellungen bewegbar ist, die in Fig. 3A und 3B gezeigt sind. Im Inneren des Gehäuses ist eine Kolbenstange 22 an ihrem einen Ende über einen quer durch das Gehäuse verlaufenden Steg 23 starr mit dem Gehäuse 20 verbunden. An ihrem anderen Ende trägt die Kolbenstange einen Kolben 24, der an den inneren Durchmesser des Zylinderkörpers 21 angepaßt ist und mit diesem den Verwirbelungsraum 2 festlegt. Wenn der Zylinderkörper 21 verschoben wird, gleitet der Kolben 24 an der Innenwand des Zylinderkörpers 21. In dem so festgelegten Verwirbelungsraum 2 ist ein zusätzlicher, flexibler Kunststoffbehälter 25 vorgesehen, der mit dem Kolben 24 gasdicht verbunden ist.

In der in Fig. 3A gezeigten Ausgangsstellung besitzt der Verwirbelungsraum 2 das kleinste Volumen. Die pulverförmige Substanz 1 befindet sich in dem flexiblen Kunststoffbehälter 25. Aus dieser Ausgangsstellung wird der Zylinderkörper 21 in die in Fig. 3B gezeigte Stellung verschoben. Während des Verschiebens verschließt ein Stopfen 26 eine im Kolben vorgesehene Öffnung 27, so daß sich aufgrund der Volumenvergrößerung im Verwirbelungsraum 2 ein Vakuum bildet und sich der flexible Kunststoffbehälter 25 ausdehnt. Mit Erreichen der in Fig. 3B gezeigten Stellung gelangt eine an der Innenwand des Zylinderkörpers angebrachte Nase 28 in Eingriff mit einem Arm eines zweiarmigen Hebels 29, an dessen anderem Arm der die Öffnung verschließende Stopfen 26 angeordnet ist. Dadurch wird die Öffnung 27 im Kolben freigegeben, so daß Luft in den evakuierten Verwirbelungsraum einströmt, die Substanz verwirbelt und das Aerosol erzeugt.

Anschließend wird der innere Zylinder 21 wieder nach oben gezogen und das Aerosol aus dem Verwirbelungsraum durch die Öffnung 27 in das Innere des Zylinders gedrückt. Am äußeren Gehäuse ist ein Mundstück 15 angebracht, durch das der Patient das erzeugte Aerosol aus dem Raum innerhalb des Zylinders 21 heraussaugen kann. Der Zylinderkörper 21 ist dazu hohl ausgebildet und besitzt an geeigneter Stelle eine Durchtrittsöffnung für das angesaugte Aerosol. Ferner besitzt der Zylinderkörper eine Lufteintrittsöffnung 31, durch die beim Inhalieren Umgebungsluft ins Innere des Zylinderkörpers gelangt. Die durch die Lufteintrittsöffnung einströmende Luft drängt das Aerosol mit der aufgewirbelten Substanz aus dem Zylinderkörper hinaus, so daß die Substanz vollständig inhaliert wird.

Der den Stopfen tragende zweiarmige Hebel 29 ist entweder so vorgespannt, daß der Stopfen 26 in seiner Ruhestellung die Öffnung 27 im Kolben verschließt und nur aufgrund der Einwirkung der Nase 28 im Inneren des Zylinderkörpers 21 oder aufgrund des Innendrucks des Verwirbelungsraumes 2 beim Zurückführen des Zylinderkörpers 21 in seine Ausgangsstellung die Öffnung 27 freigibt, oder er wird mit Hilfe eines Fadens 32 kurz vor Erreichen der Ausgangsstellung in die verschließende Position gebracht.

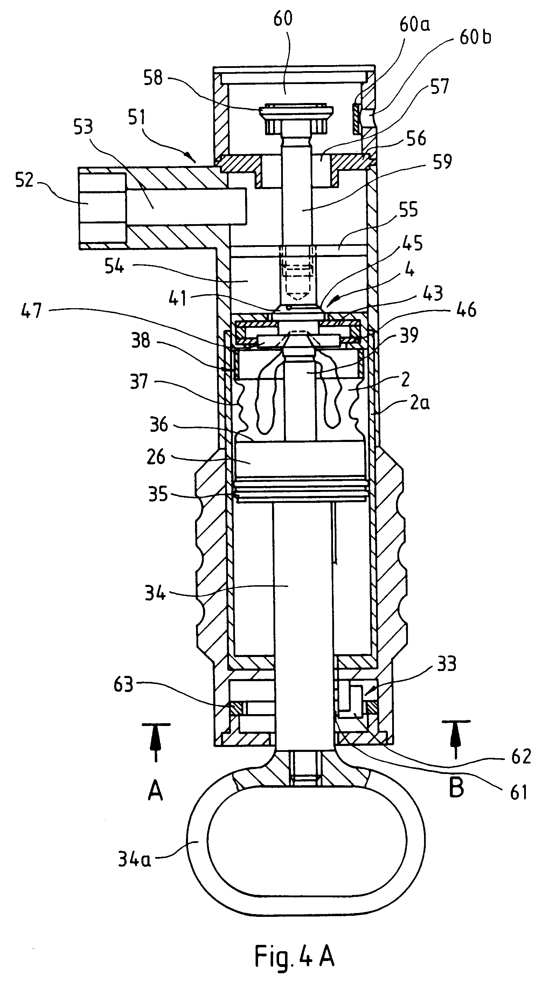

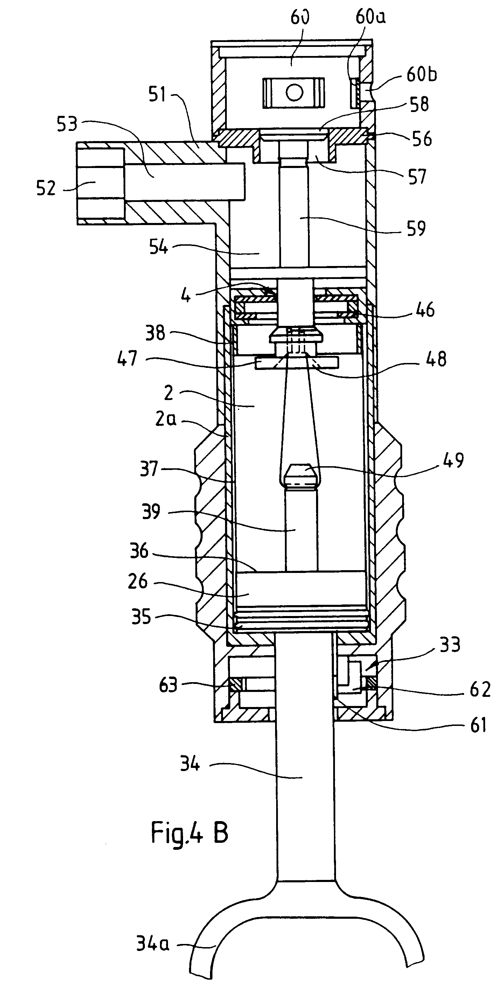

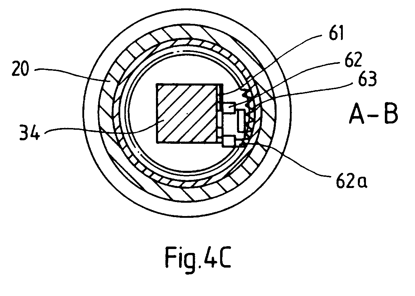

Die Fig. 4A bis 4C zeigen eine äußerst bevorzugte Ausgestaltung einer erfindungsgemäßen Vorrichtung zur Erzeugung eines Aerosols zur Durchführung des erfindungsgemäßen Verfahrens. Gleiche Teile sind mit gleichen Bezugszeichen versehen wie bei den vorangehenden Figuren.

Die Vorrichtung weist in einem Zylinder 2a einen Verwirbelungsraum 2 auf, in dem auch die Gesamtmenge der zu verwirbelnden Substanz untergebracht ist. Die Volumenänderung des Verwirbelungsraums 2 erfolgt durch einen Kolben 2b, der in dem Zylinder 2a verschiebbar ist. Auf der dem Kolben 2b gegenüberliegenden Seite des Verwirbelungsraumes ist ein Verschlußorgan 4 in Form eines Ventils vorgesehen.

Der Kolben 2b ist über eine aus dem Zylinder 2a heraus- und durch ein weiter unten zu erläuterndes Zählwerk 33 ragende Kolbenstange 34 mittels eines mit dieser verbundenen Griffes 34a im Zylinder 2a verschiebbar.

Der Kolben ist mit Dichtungen 35 versehen. Mit seiner zum Verwirbelungsraum 2 reichenden Stirnseite 36 ist ähnlich wie bei der Ausgestaltung der Fig. 3A, 3B eine flexible Hülle 37 in Form einer Latexmembran oder dergleichen verbunden, die weiter im Bereich des Verschlußorgans 4 bei 38 an der Wandung des Zylinders 2a festgelegt ist. Der Verwirbelungsraum 2 ist innerhalb der Hülle 37 ausgebildet. Die Substanz 1 befindet sich ebenfalls innerhalb der Hülle. Hierdurch wird verhindert, daß sich Substanzmaterial an der Innenwandung des Zylinders 2a festsetzt und die Bewegung des Kolbens 2b erschwert oder sonstwie beeinträchtigt.

Über die dem Verwirbelungsraum 2 zugewandte Stirnseite 36 des Kolbens 2b ragt in den Verwirbelungsraum 2 ein Stößel 39, dessen Funktion weiter unten erläutert wird.

Das Verschlußorgan 4 weist einen beweglichen Ventilkörper 41 und einen Ventilsitz 42 auf. Der Ventilsitz 42 wird durch einen Dichtring 43 gebildet, der am Ventilkörper 41 im Schließzustand dicht aufliegt. Auf der dem Verwirbelungsraum 2 abgewandten Seite ist der Ventilkörper 41 mit einer konusförmigen Fläche 45 versehen, die beim erneuten Schließen des Ventils des Verschlußorgans 4 an der Dichtring 43 entlanggleitet.

Die Innenseite des Dichtungsringes ist vorzugsweise nicht zylinderförmig, sondern schneidenförmig oder abgerundet ausgebildet.

Mit Abstand zur Dichtung 43 in Richtung auf den Verwirbelungsraum 2 hin ist ein ebenfalls elastischer Abstreifring 46 am Zylinder 2a festgelegt, der mit einem mit dem Ventilkörper 41 ebenfalls zum Verwirbelungsraum 2 hin verbundenen, flachen Zylinderkörper 47 mit gegenüber dem Ventilkörper 41 vergrößerten Durchmeseser zusammenwirkt. Ventilkörper 41 und Zylinderkörper 47 können einstückig ausgebildet sein.

Im Zylinderkörper 47 ist eine zentralsymmetrische, kegelstumpfförmige Ausnehmung 48 ausgebildet, die mit einem kegelstumpfförmigen, vorderen Ende 49 des Stößels 39 in noch zu beschreibender Weise zusammenwirkt.

Mit dem Zylinder 2a ist ein Mundstück 51 verbunden, dessen Haupterstreckungsrichtung sich senkrecht zu der des Zylinders 2a erstreckt. Das Mundstück weist einen zu einer Auslaßöffnung 52 führenden Auslaßkanal 53 auf.

Zwischen Auslaßbereich 54 des Verwirbelungsraumes 2 hinter dem Verschlußorgan 4 und dem Auslaßkanal 53 ist eine Siebplatte 55 angeordnet, durch die das aus dem Verwirbelungsraum 2 herausgedrückte Aerosol in den Kanal 53 und von diesem aus der Öffnung 52 herausgedrückt wird, wobei die Aerosolströmung gleichgerichtet, d.h. in eine im wesentlichen laminare Strömung gebracht wird und durch die Verwirbelung im Verwirbelungsraum 2 entstandene Turbulenzen des Aerosols weitgehend unterdrückt werden.

In einer dem Verschlußorgan 4 abgewandten Trennwand 56 des Mundstücks 51 ist vorzugsweise fluchtend mit dem Verschlußorgan 4 eine Öffnung 57 ausgebildet, die durch eine Verschlußplatte 58 verschließbar ist, die über einen axialen Ansatz 59 des Ventilkörpers 41 beim Öffnen desselben verschließbar ist und bei geschlossenem Verschlußorgan 4 geöffnet ist.

Die Öffnung 57 mündet in einen Zwischenraum 60, der über auf ihrer Innenseite durch als Rückschlagventile 60a wirkende Klappen abdeckbare Öffnungen 60b in der Zylinderwand 2a mit der Umgebung in Verbindung steht. So kann über die Öffnungen 60b und die Öffnung 57 nur Luft eingesaugt, aber nicht herausgedrückt werden.

Wie schon oben erwähnt, ist an dem dem Mundstück 51 abgewandten Ende des Zylinders 2a ein Zählwerk 33 vorgesehen. Hierzu ist an der Kolbenstange 34 eine federnde Nase 61 ausgebildet, die auf ein Ritzel 62 wirkt, dessen Achse senkrecht zur Achse der Kolbenstange 34 ausgerichtet ist. An der abgewandten Stirnseite des Ritzels 62 ist eine Schnecke 62a ausgebildet, die in einen großen Zahnkranz 63 mit innenliegender Verzahnung eingreift, dessen Achse parallel zur Achse der Kolbenstange 34 ausgerichtet ist und gegebenenfalls mit dieser zusammenfällt. An der Außenseite des Zahnkranzes 63 sind Markierungen angeordnet, die durch ein Fenster erkennbar sind.

Alternativ könnte ein Zahnriemen vorgesehen sein, der in gleicher Weise über einen federnden Ansatz an der Kolbenstange 34 gerichtet betätigbar ist und Farbmarkierungen aufweist, die durch ein Fenster festgestellt werden können. So kann in beiden Fällen festgestellt werden, ob die vorgesehene Anzahl von Betätigungen ausgeführt und damit die in der Vorrichtung ursprünglich eingefüllte Substanz verbraucht ist.

Die erfindungsgemäße Vorrichtung funktioniert folgendermaßen:

Der Kolben 2b wird aus der in der Fig. 4A dargestellten Position, bei der er höchstens soweit nach oben, d.h. in Richtung auf das Mundstück 51, zugeschoben ist, daß die Spitze 49 des Ansatzes 39 in die konische Ausnehmung 48 der Platte 47 ragt, mittels des Griffes 33 nach unten, d.h. von dem Mundstück 51 fortgezogen. Das Verschlußorgan 4 ist dabei geschlossen. Hierdurch wird der Verwirbelungsraum 2 mit einem Unterdruck, einem Vakuum versehen.

Kurz bevor der Kolben 2b an der dem Mundstück 51 abgewandten Stirnseite des Zylinders 2a angelangt, befindet sich der zwischen dem Ventilkörper 41 und dem Ansatz 39 des Kolbens 2b befestigte zugfeste und auch gegen Zug relativ unelastische Faden 50 in seiner gespannten Stellung. Ein weiteres Herunterziehen des Kolbens 2b bis zur dem Mundstück 51 abgewandten Stirnseite bewirkt, daß das Verschlußorgan 4 durch Herunterziehen des Ventilkörpers 41 geöffnet wird (Fig. 4B). Gleichzeitig wird die Öffnung 57 durch die Verschlußplatte 58 verschlossen.

Durch das geöffnete Verschlußorgan 4 kann nun über die Öffnung 52 des Mundstücks Luft aufgrund des Unterdrucks des Verwirbelungsraums 2 in diesen einströmen, wodurch die in diesem befindliche Substanz 1 verwirbelt wird und damit ein Aerosol entsteht.

Im folgenden wird mittels des Griffes 33 der Kolben 2b wieder auf das Mundstück 51 zugedrückt. Hierbei wird zunächst das im Verwirbelungsraum 2 gebildete Aerosol aus dem Verwirbelungsraum heraus und durch die Siebplatte 55 in den Kanal 53 des Mundstücks 51 gedrückt, bis die vordere Spitze 49 des Kolbenansatzes 39 in der Ausnehmung 48 der Platte 47 des Verschlußorgans 4 eingreift und das Verschlußorgan 4 wieder schließt. Gleichzeitig wird die Öffnung 57 geöffnet, indem die Verschlußplatte 58 von der Öffnung abgehoben wird.

Solange die Öffnung 57 geschlossen ist und damit das Verschlußorgan 4 geöffnet ist, kann der Benutzer der erfindungsgemäßen Vorrichtung nicht einatmen. Es wird damit verhindert, daß er eine undefinierte Menge an Aerosol aus dem Verwirbelungsraum 2 durch Einatmen einsaugt.

Dadurch, daß während des Ausdrückens von Aerosol aus dem Verwirbelungsraum 2 und der Öffnungszeit des Verschlußorgans 4 die Öffnung 57 geschlossen ist, wird damit eine genau definierte Menge Aerosol aus dem Verwirbelungsraum 2 in das Mundstück 51 eingebracht, die durch den Hub des Kolbens 2b bestimmt ist. Wäre in diesem Zeitbereich die Öffnung 57 geöffnet, so könnte im Gegenstrom Luft durch die Öffnung 57 und das geöffnete Verschlußorgan 4 in den Verwirbelungsraum 2 einströmen, so daß ein Benutzer durch Einatmen eine undefinierte Menge Aerosol aus dem Verwirbelungsraum 2 einsaugen könnte.

Nachdem, wie beschrieben, die Öffnung 57 geöffnet wurde, kann der Benutzer das im Kanal 53 des Mundstücks 51 befindliche Aerosol inhalieren. Dabei kann Luft über die Öffnung 60b, den Raum 60 und die Öffnung 57 nachströmen, da sich beim Ansaugen die Klappen 60a von den Öffnungen 60b abheben und diese freigeben (wiederum Fig. 4A). Anschließend steht die erfindungsgemäße Vorrichtung zu einer erneuten Benutzung bereit.

## Patentansprüche

1. Verfahren zum Erzeugen eines Aerosols aus einer pulverförmigen Substanz (1), insbesondere zur Inhalation, dadurch gekennzeichnet, daß in einem Verwirbelungsraum (2) zunächst ein Unterdruck in Gegenwart der pulverförmigen Substanz (1) erzeugt wird, daß anschließend in den Verwirbelungsraum (2) Gas derart einströmt, daß die darin enthaltene pulverförmige Substanz (1) verwirbelt und ein Aerosol (5) erzeugt wird und unmittelbar anschließend Aerosol (5) aus dem dieses enthaltenden Verwirbelungsraum (2) ausgebracht wird.

2. Verfahren nach Anspruch 1, dadurch gekennzeichnet, daß im Verwirbelungsraum (2) ein Druck von maximal 100 Pa, vorzugsweise weniger als 50 Pa erzeugt wird.

3. Verfahren nach Anspruch 1, dadurch gekennzeichnet, daß der Unterdruck in dem die Substanz (1) enthaltenden Verwirbelungsraum (2) durch Vergrößern des Verwirbelungsraumes (2) erzeugt wird.

4. Verfahren nach Anspruch 1, dadurch gekennzeichnet, daß der Unterdruck in dem die Substanz (1) enthaltenden Verwirbelungsraum (2) durch Absaugen, vorzugsweise mittels einer motorisch angetriebenen Pumpe, hergestellt wird.

5. Verfahren nach einem der Ansprüche 1 bis 4, dadurch gekennzeichnet, daß das Aerosol (5) aus dem das Aerosol (5) enthaltenden Verwirbelungsraum (2) durch Herausdrücken ausgebracht wird.

6. Verfahren nach einem der vorangehenden Ansprüche, dadurch gekennzeichnet, daß die Verwirbelung in einem den Verwirbelungsraum (2) bildenden Vorratsraum für die Substanz (1) erfolgt.

7. Verfahren nach einem der Ansprüche 1 bis 5, dadurch gekennzeichnet, daß eine definierte Menge der Substanz in den Verwirbelungsraum (2) zur Verwirbelung derselben eingebracht wird.

8. Verfahren nach einem der vorangehenden Ansprüche, dadurch gekennzeichnet, daß ein mit dem Verwirbelungsraum (2) verbindbares Mundstück (51) während des Eindrückens des Aerosols (5) in das Mundstück (51) bis gegebenenfalls auf einen Auslaß des Mundstücks (51) für das spätere Einatmen durch den Patienten gegenüber der Umgebung absperrbar ist.

9. Verfahren nach einem der vorangehenden Ansprüche, dadurch gekennzeichnet, daß ein mit dem Verwirbelungsraum (2) verbindbares Mundstück (51) nach Eindrücken des Aerosols (5) in das Mundstück (51) auch bei an diesem angesetztem Mund des Benutzers zur Umgebung geöffnet ist.

10. Verfahren nach einem der vorangehenden Ansprüche, dadurch gekennzeichnet, daß das Aerosol (5) weitgehend laminar in das Mundstück (51) strömt.

11. Vorrichtung zur Erzeugung eines Aerosols aus einer pulverförmigen Substanz (1), insbesondere zur Inhalation, mit einem Vorratsraum und mit einem Verwirbelungsraum (2), in dem ein Unterdruck erzeugbar ist, der in seinem Inneren zumindestens die zu verwirbelnde Substanz (1) aufnimmt, in den durch eine Öffnung (3; 11,12; 31; 57) ein Gas zum Verwirbeln der Substanz (1) und zur Erzeugung eines Aerosols (5) einbringbar ist und aus dem durch eine Öffnung (3; 11,12; 30; 57) dieses ausbringbar ist, dadurch gekennzeichnet, daß die Öffnungen (3; 11,12; 30, 31; 57) verschließbar sind, daß der Verwirbelungsraum (2) im Vorratsraum für die Substanz (1) ausgebildet ist, in dem die Gesamtmenge der Substanz (1) untergebracht ist und aus dem eine genau definierte Teilmenge der Substanz (1) als Aerosol (5) durch die Öffnung (3; 11,12; 30; 57) zum Ausbringen des Aerosols (5) ausbringbar ist.

12. Vorrichtung nach Anspruch 11, gekennzeichnet durch eine Pumpe zur Erzeugung eines Unterdrucks.

13. Vorrichtung nach Anspruch 11, dadurch gekennzeichnet, daß der Verwirbelungsraum (2) in abgeschlossenem Zustand zur Herstellung des Unterdrucks in ihm vergrößerbar ist.

14. Vorrichtung nach Anspruch 13, dadurch gekennzeichnet, daß nach Öffnen der Öffnung das Gas in den Verwirbelungsraum (2) aufgrund des in diesem erzeugten Unterdrucks einströmt.

15. Vorrichtung nach einem der Ansprüche 13 bis 14, dadurch gekennzeichnet, daß der Verwirbelungsraum (2) von einer Anordnung aus einem Kolben (2b; 24) und einem Zylinder (2a; 21) gebildet ist, bei der der Verwirbelungsraum (2) durch Verschieben des Kolbens (2b; 24) gegenüber dem Zylinder (2a; 21) vergrößerbar ist.

16. Vorrichtung nach einem der Ansprüche 11 bis 15, dadurch gekennzeichnet, daß zwischen dem Innenraum (53) eines Mundstücks (51, 15) und der Umgebung eine verschließbare Öffnung (57) vorgesehen ist, die beim Öffnen der Austrittsöffnung (3) des Verwirbelungsraums (2) zum Ausströmen des Aerosols aus dem Verwirbelungsraum (2) geschlossen ist.

17. Vorrichtung nach einem der Ansprüche 11 bis 16, dadurch gekennzeichnet, daß zwischen dem Inneren (53) eines Mundstücks (51, 15) und der Umgebung eine verschließbare Öffnung (57) vorgesehen ist, die nach Schließen der Austrittsöffnung (3) des Verwirbelungsraumes (2) geöffnet ist.

18. Vorrichtung nach Anspruch 16 oder 17, dadurch gekennzeichnet, daß ein Verschlußorgan (4) für die Austrittsöffnung (3) des Verwirbelungsraums (2) und ein Verschlußteil (58) für die Öffnung (27) zwischen dem Inneren (53) des Mundstücks (51) und der Umgebung miteinander verbunden sind.

19. Vorrichtung nach Anspruch 18, dadurch gekennzeichnet, daß Verschlußorgan (4) und Verschlußteil (58) starr miteinander verbunden sind.

20. Vorrichtung nach einem der Ansprüche 11 bis 19, dadurch gekennzeichnet, daß zwischen der Austrittsöffnung (3) des Verwirbelungsraums (2) und dem Inneren (23) eines Mundstücks (51) ein Strömungsgleichrichter, z.B. eine Sieb- oder Lochplatte, angeordnet ist.

21. Vorrichtung nach einem der Ansprüche 11 bis 20, dadurch gekennzeichnet, daß das Verschlußteil (4) für die Austrittsöffnung (3) des Verwirbelungsraumes (2) mit einem in diesem bewegbaren Kolben (2b) durch einen Faden verbunden ist.

22. Vorrichtung nach einem der Ansprüche 11 bis 21, dadurch gekennzeichnet, daß ein das Volumen des Verwirbelungsraumes (2a) verändernder Kolben (2b) mit einem mit einem Verschlußorgan (4) einer Austrittsöffnung (3) des Verwirbelungsraumes (2a) zusammenwirkenden Stößel (39) zum Verschließen der Austrittsöffnung (3a) verbunden ist.

## Claims

1. Process for the generation of an aerosol from a powdered substance (1), especially for inhaling, characterized in that a negative pressure is first generated in the presence of the powdered substance (1) in a dispersion volume (2), that subsequently gas flows in the dispersing volume (2) in such a way, that the powdered substance (1) contained therein disperses and aerosol (5) is generated, and aerosol (5) is brought out of the dispersion volume (2) containing the same immediately thereafter.

2. Process according to claim 1, characterized in that a maximum pressure of 100 Pa, preferably less than 50 Pa, is generated in the dispersion volume (2).

3. Process according to claim 1, characterized in that the negative pressure is generated in the dispersion area (2) that contains the substance (1) through enlargement of the dispersion volume (2).

4. Process according to claim 1, characterized in that the negative pressure is generated in the dispersion volume (2) that contains the substance (1) by suction, preferably by means of a motor-driven pump.

5. Process according to any of the claims 1 to 4, characterized in that the aerosol (5) is brought out of the dispersion volume (2) which contains the aerosol (5) by expulsion.

6. Process according to any of the preceding claims, characterized in that the dispersion occurs in a storeroom for the substance (1) forming the dispersion volume (2).

7. Process according to any of the claims 1 to 5, characterized in that a defined amount of the substance is brought in the dispersion volume (2) for its dispersion.

8. Process according to any of the preceding claims, characterized in that a mouthpiece (51) attachable to the dispersion volume (2) can be blocked off against the surroundings during the influx of the aerosol (5) in the mouthpiece (51), optionally except for the outlet of the mouthpiece (51) for the later inhalation by the patients.

9. Process according to any of the preceding claims, characterized in that a mouthpiece (51) attachable to the dispersion volume (2) is open to the surroundings after influx of the aerosol (5) in the mouthpiece (51) also when the mouth of the user is on the mouthpiece.

10. Process according to any of the preceding claims, characterized in that the aerosol (5) flows in a substantially laminar manner in the mouthpiece (51).

11. Device for the generation of an aerosol from a powdered substance (1), especially for inhaling, with a storeroom and with a dispersion volume (2), in which a negative pressure can be generated, which takes in at least the dispersed substance (1) in its interior, into which a gas can be brought in for the dispersion of the substance (1) anf for the generation of aerosol (5) through an opening (3; 11, 12; 31; 57) and from which it can be brought out through an opening (3; 11, 12; 30; 57), characterized in that the openings (3; 11, 12; 30; 31; 57) are closable, that the dispersion volume (2) is formed in the storeroom for the substance (1), which comprises the total amount of the substance (1) and from which a specifically defined amount of the substance (1), as aerosol (5), can be brought out through the opening (3; 11, 12; 30; 57) for the release of the aerosol (5).

12. Device according to claim 11, characterized by a pump for the generation of a negative pressure.

13. Device according to claim 11, characterized in that in closed condition the dispersion volume (2) is expandable to produce the negative pressure in the area.

14. Device according to claim 13, characterized in that the gas flows into the dispersion volume (2) after opening of the entrance due to the negative pressure generated therein.

15. Device according to one of the claims 13 or 14, characterized in that the dispersion volume (2) is built by an arrangement of a piston (2b; 24) and a cylinder (2a; 21), wherein the dispersion volume (2) is expandable through the movement of the piston (2b; 24) against the cylinder (2a; 21).

16. Device according to any of the claims 11 to 15, characterized in that between the interior area (53) of a mouthpiece (51, 15) and the surroundings a closable opening (57) is provided, which is closed by the opening of the exit opening (3) of the dispersion volume (2) for the outflow of the aerosol out of the dispersion volume (2).

17. Device according to any of the claims 11 to 16, characterized in that between the interior (53) of a mouthpiece (51, 15) and the surroundings a closable opening (57) is provided, which is open after the closure of the exit opening (3) of the dispersion volume (2).

18. Device according to one of the claims 16 or 17, characterized in that a closure device (4) for the exit opening (3) or the dispersion volume (2) and a closure unit (58) for the opening (27) between the interior (53) of the mouthpiece (51) and the surroundings are connected to one another.

19. Device according to claim 18, characterized in that closure device (4) and closure unit (58) are immovably connected to one another.

20. Device according to any of the claims 11 to 19, characterized in that between the exit opening (3) of the dispersion volume (2) and the interior (23) of a mouthpiece (51) a flow regulator, e.g. a sieve or a perforated plate, is arranged.

21. Device according to any of the claims 11 to 20, characterized in that the closure unit (4) for the exit opening (3) of the dispersion volume (2) is connected to a movable piston (2b) therein through a cord.

22. Device according to any of the claims 11 to 21, characterized in that a piston (2b) that changes the volume of the dispersion volume (2a) is connected to a plunger (39) which works together with a closure device (4) of an exit opening (3) of the dispersion volume (2a) for the closure of the exit opening (3a).

## Revendications

1. Procédé de production d'un aérosol à partir d'une matière (1) pulvérulente, plus particulièrement pour l'inhalation, caractérisé en ce que l'on génère d'abord une dépression dans une chambre de turbulence (2) en présence de la matière (1) pulvérulente, en ce que l'on introduit ensuite un fluide gazeux dans la chambre de turbulence (2) de façon que la matière (1) pulvérulente qui y est contenue soit soumise à turbulence et produise un aérosol (5), et en ce qu'immédiatement après, on évacue l'aérosol (5) de la chambre de turbulence qui le contient.

2. Procédé selon la revendication 1, caractérisé en ce que dans la chambre de turbulence (2), on génère une pression de 100 Pa au maximum, mais de préférence inférieure à 50 Pa.

3. Procédé selon la revendication 1, caractérisé en ce que la dépression dans la chambre de turbulence (2) contenant la matière (1) est générée par agrandissement de cette chambre (2).

4. Procédé selon la revendication 1, caractérisé en ce que la dépression dans la chambre de turbulence (2) contenant la matière (1) est générée par aspiration, de préférence avec une pompe actionnée par moteur.

5. Procédé selon l'une quelconque des revendications 1 à 4, caractérisé en ce que l'on évacue par pression l'aérosol (5) de la chambre de turbulence (2) le contenant.

6. Procédé selon l'une quelconque des revendications précédentes, caractérisé en ce que la turbulence se produit dans une chambre-réservoir conformée pour la matière (1) constituant la chambre de turbulence (2).

7. Procédé selon l'une quelconque des revendications 1 à 5, caractérisé en ce que l'on introduit une quantité prédéfinie de matière dans la chambre de turbulence (2) en vue de la turbulence.

8. Procédé selon l'une quelconque des revendications précédentes, caractérisé en ce que l'on peut obturer par rapport au milieu ambiant un embout (51) en liaison avec la chambre de turbulence (2) pendant l'introduction de l'aérosol (5) dans l'embout (51) jusqu'à la sortie, le cas échéant, de l'embout (51) pour une inhalation ultérieure par le patient.

9. procédé selon l'une quelconque des revendications précédentes, caractérisé en ce qu'un embout (51) en liaison avec la chambre de turbulence (2) est ouvert au milieu ambiant après introduction de l'aérosol (5) dans l'embout (51) même lorsqu'il est en contact avec la bouche de l'utilisateur.

10. Procédé selon l'une quelconque des revendications précédentes, caractérisé en ce que l'aérosol (5) pénètre dans l'embout (51) de façon essentiellement laminaire.

11. Dispositif de production d'un aérosol à partir d'une matière (1) pulvérulente, en particulier pour l'inhalation, comprenant une chambre-réservoir et une chambre de turbulence (2) dans laquelle on peut générer une dépression, qui contient au moins la matière (1) à soumettre à turbulence, dans laquelle on peut injecter par une ouverture (3 ; 11,12 ; 31 ; 57) un gaz pour soumettre la matière (1) à turbulence et produire un aérosol (5), et dont on peut éjecter cet aérosol par une ouverture (3; 11,12 ; 30 ; 57), caractérisé en ce que les ouvertures (3 ; 11,12 ; 30 ; 31 ; 57) sont obturables, en ce que la chambre de turbulence (2) est conformée dans la chambre-réservoir pour la matière (1), dans laquelle la quantité totale de matière (1) est introduite et dont on peut éjecter une quantité partielle définie avec précision de la matière (1) sous forme d'aérosol (5) à travers l'ouverture (3 ; 11,12 ; 30 ; 57) pour la diffusion de l'aérosol (5).

12. Dispositif selon la revendication 11, caractérisé en ce que la génération d'une dépression est réalisée par une pompe.

13. Dispositif selon la revendication 11, caractérisé en ce qu'en position obturée, la chambre de turbulence (2) peut être agrandie pour y générer la dépression.

14. Dispositif selon la revendication 13, caractérisé en ce qu'après ouverture du passage, le gaz pénètre dans la chambre de turbulence (2) en raison de la dépression qui y est générée.

15. Dispositif selon l'une quelconque des revendications 13 à 14, caractérisé en ce que la chambre de turbulence (2) est formée par une structure constituée d'un piston (2b ; 24) et d'une chemise (2a ; 21), structure dans laquelle la chambre de turbulence (2) peut être agrandie par déplacement du piston (2b ; 24) par rapport à la chemise (2a ; 21).

16. Dispositif selon l'une quelconque des revendications 11 à 15, caractérisé en ce qu'entre l'espace interne (53) d'un embout (51,15) et le milieu ambiant est prévue une ouverture (57) obturable, qui est fermée pendant l'ouverture de la sortie (3) de la chambre de turbulence (2) pour l'évacuation de l'aérosol hors de celle-ci.

17. Dispositif selon l'une quelconque des revendications 11 à 16, caractérisé en ce qu'entre l'espace interne (53) d'un embout (51,15) et le milieu ambiant est prévue une ouverture (57) obturable, qui est ouverte après la fermeture de la sortie (3) de la chambre de turbulence (2).

18. Dispositif selon l'une quelconque des revendications 16 ou 17, caractérisé en ce qu'un organe d'obturation (4) pour la sortie (3) de la chambre de turbulence (2) et une pièce d'obturation (58) pour l'ouverture (27) entre l'espace interne (53) de l'embout (51) et le milieu ambiant sont reliés entre eux.

19. Dispositif selon la revendication 18, caractérisé en ce que l'organe d'obturation (4) et la pièce d'obturation (58) sont reliés entre eux de façon rigide.

20. Dispositif selon l'une quelconque des revendications 11 à 19, caractérisé en ce qu'entre la sortie (3) de la chambre de turbulence (2) et l'espace interne (23) d'un embout (51) est disposé un régulateur d'écoulement, par exemple une plaquette perforée.

21. Dispositif selon l'une quelconque des revendications 11 à 20, caractérisé en ce que l'organe d'obturation (4) pour la sortie (3) de la chambre de turbulence (2) est relié par un fil à un piston (2b) déplaçable dans cette chambre.

22. Dispositif selon l'une quelconque des revendications 11 à 21, caractérisé en ce qu'un piston (2b) modifiant le volume de la chambre de turbulence (2a) est relié à une tige-poussoir (39) coopérant avec un organe d'obturation (4) d'une sortie (3) de la chambre de turbulence (2a) pour l'obturation de la sortie (3a).
